(19) Europäisches Patentamt

European Patent Office

Office européen des brevets

(11) **EP 1 485 144 B1**

(12) **EUROPEAN PATENT SPECIFICATION**

(45) Date of publication and mention
of the grant of the patent:
**27.07.2005   Bulletin 2005/30**

(51) Int Cl.⁷: **A61M 1/10**

(86) International application number:
**PCT/CA2003/000103**

(21) Application number: **03700754.9**

(22) Date of filing: **27.01.2003**

(87) International publication number:
**WO 2003/075981 (18.09.2003 Gazette 2003/38)**

(54) **DUAL INLET MIXED-FLOW BLOOD PUMP**

BLUTPUMPE MIT AXIALEM UND RADIALEM DURCHFLUSS UND MIT DOPPELTEM EINGANG

POMPE SANGUINE HELICOCENTRIFUGE A DOUBLE CANAL D'ENTREE

(84) Designated Contracting States:
**AT BE BG CH CY CZ DE DK EE ES FI FR GB GR HU IE IT LI LU MC NL PT SE SI SK TR**

(30) Priority: **08.03.2002   CA 2374989**

(43) Date of publication of application:
**15.12.2004   Bulletin 2004/51**

(73) Proprietor: **Cardianove Inc.**
**Montreal, Quebec H3S 2V6 (CA)**

(72) Inventors:
• **CARRIER, Michel**
**Montreal, Quebec H1M 3H9 (CA)**
• **GARON, André**
**Ville D'Anjou, Quebec H1K 3V7 (CA)**
• **CAMARERO, Ricardo**
**Verdun, Quebec H3E 1N4 (CA)**
• **PELLETIER, Conrad**
**Montreal, Quebec H3S 2V6 (CA)**

(74) Representative: **Thébault, Jean-Louis**
**SCHMIT-CHRETIEN-SCHIHIN**
**111, cours du Médoc**
**33300 Bordeaux (FR)**

(56) References cited:
**EP-A- 0 847 767         WO-A-98/53864**
**WO-A-99/34847         US-A- 4 817 586**
**US-A- 5 588 812         US-A- 5 613 935**
**US-A- 5 851 174         US-B1- 6 176 848**

**Description**

## FIELD OF THE INVENTION

The present invention relates to a mixed flow blood pump displaying characteristics of both radial-flow and axial-flow pumps.

## BACKGROUND OF THE INVENTION

In North America, heart related diseases are still the leading cause of death. Among the causes of heart mortality are congestive heart failure, cardiomyopathy and cardiogenic shock. The incidence of congestive heart failure increases dramatically for people over 45 years of age. In addition, a large part of the population in North America is now entering this age group. Thus, the people who will need treatment for these types of diseases comprise a larger segment of the population. Many complications related to congestive heart failure, including death, could be avoided and many years added to these persons' lives if proper treatments were available.

The types of treatment available for patients of heart failure depend on the extent and severity of the illness. Many patients can be cured with rest and drug therapy but there are still severe cases that require various heart surgery, including heart transplantation. Actually, the mortality rate for patients with cardiomyopathy who receive drug therapy is about 25 % within two years and still is some form of these diseases that cannot be treated medically. One of the last options that remain for these patients is heart transplantation. Unfortunately, according to the procurement agency UNOS (United Network for Organ Sharing in the United States), the waiting list for heart transplantation grows at a rate of more than twice the number of heart donors.

Considering these facts, it appears imperative to offer alternative treatments to heart transplantation. The treatment should not only add to a recipient's longevity but also improve his quality of life. In this context, mechanical circulatory support through Ventricular Assist Devices (VAD) is a worthwhile alternative given the large deficiency in the number of available organ donors. In the 1980's, successful experiments with mechanical hearts and VADs serving as a bridge to transplantation increased significantly. The accumulated knowledge in all aspects of patient care, device designs and related problems led to the use of VADs as permanent implants. Now, it appears appropriate to address the problem of end stage heart failure with permanent mechanical heart implants. Among the various mechanical support devices, axial-flow VADs with a projected life span of five to ten years provides a very interesting approach. It is estimated that eight thousand (8,000) patients per year in Canada and seventy-six thousand patients (76,000) per year in the United States could benefit from VADs.

In 1980, the National Heart, Lung and Blood Institute (NHLBI) of the United States defined the characteristics for an implantable VAD (Altieri, F.D. and Watson, J.T., 1987, "Implantable Ventricular Assist Systems", *Artif Organs,* Vol. 11, pp. 237-246). These characteristics include medical requirements including restoration of hemodynamic function (pressure and cardiac index) avoidance of hemolysis, prevention of clot formation, infection and bleeding, and minimisation of the anti-coagulation requirement. Further technical requirements include: small size, control mode, long life span (> 2 years), low heating, noise and vibration.

VADs can be used in several circumstances where a patient has poor hemodynamic functions (low cardiac output, low ejection fraction, low systolic pressure). Whatever the origin of the cardiac failure, the goal of the VAD is to help the heart in its pumping action. The VAD reduces the load on the heart by enhancing circulation, thus restoring the hemodynamic functions and providing improved end organ perfusion. Many current VAD devices can achieve these goals; however they are not optimal, and hemolysis and thrombus formation are still important problems requiring investigation.

In the 1970's, the first approach to the problem of mechanical support was to imitate as much as possible the heart physiology. This resulted in the development of several pulsatile devices, some of these initial designs being still in use. The first developments were pneumatically driven devices while a second generation of pumps was electrically actuated. In the 1990's, a new generation of pumps has emerged which addresses certain problems associated with previous devices (size and power consumption). These pumps are non-pulsatile devices divided into two main categories: radial-flow blood pumps, and axial-flow blood pumps.

In a non-pulsatile VAD, an impeller is enclosed in a housing and continuously rotates to produce a pumping action. The faster the rotation, the higher the blood flow. These devices are called non-pulsatile or continuous because they provide for a constant blood flow. Most axial-flow blood pumps operate around 10 000 RPM (Rotations Per Minute). However, in in-vivo conditions, there is a dynamic range (about 1000 RPM around the operating point) over which the output flow is pulsatile. Since the native heart is still contracting, a pressure difference between the ventricle (inlet) and the aorta (outlet) is created. This pressure variation will produce a variation in the pump flow. The range of rotational speed over which pulsatile flow occurs is small; at lower speed back flow is observed (in diastole) and at higher speed the load on the heart is reduced to zero. In the latter case, no pressure variation occurs resulting in non-pulsatile flow.

Many advantages are associated with the use of non-pulsatile VADs and they all have a strong impact on the physiology as well as on the clinical management. These advantages include:

**Size:** Non-pulsatile VADs provide a much smaller volume than pulsatile VADs, around 25 cc for an axial-flow blood pump, and 100 cc for a radial-flow blood pump, compared to 150 cc and more for pulsatile devices. For the sake of comparison a complete axial-flow VAD is usually smaller than the graft used for pulsatile pump. The clinical impact is the possibility to use this type of VADs in small adults as well as in children. Also, the small dimensions allow for placement of the pump in a more orthotopic position; that is, in the thorax near the heart instead of the upper abdomen. This eliminates the use of long cannula passing through the diaphragm. Furthermore, for axial-flow VADs, the shape and size can be selected allowing for placement of the VAD in an intra-ventricular position.

**Power:** The electrical power required to drive a non-pulsatile VAD is lower than for pulsatile VADs.

**Simplicity:** Non-pulsatile VADs are mechanically simpler than pulsatile VADs: they do not require complex structures such as valves, diaphragms, blood sacs, vents or compliance chambers. Non-pulsatile continuous VADs are made of a simple motor to which is coupled an impeller contained in a housing. One important advantage of a simple mechanical design is its extended durability. Durability as long as five to ten years (Nosé, Y., 1995a, "Can We Develop a Totally Implantable Rotary Blood Pump? ", *Artificial Organs*, Vol. 19, pp. 561-562; and Jarvik, R.K., 1995, "System Consideration Favoring Rotary Artificial Hearts with Blood-Immersed Bearing", *Artificial Organs,* Vol. 19, pp. 565-570) could be achieved with continuous VADs compared with two years for pulsatile VADs (Pierce, W.S., Sapirstein, J.S. and Pae, W.E., 1996, "Total Artificial Heart: From Bridge to Transplant to Permanent Use", *Ann Thorac Surg,* Vol. 61, pp. 342-346). In principle, this would allow not only to use a non-pulsatile VAD as a bridge to transplantation but also as long term mechanical support.

**Hemolysis:** Hemolysis, or tearing of the red blood cells, can be estimated *in vitro* with a parameter called the Normalised Index of Hemolysis (NIH).

**Infection:** Interestingly, the probability of infection is reduced with continuous VADs. This is due in large part to the transcutaneous vent of a pulsatile VAD which is an open door for opportunist infections and therefore requires daily cleaning.

**Patient Issues:** Non-pulsatile VADs require less maintenance allowing the patient a greater autonomy. Also, most patients with a VAD are discharged from the hospital and returned to a normal life after about a month. Presently, because of the vent in pulsatile VADs, patients cannot take a bath or swim since water could enter the motor compartment. Continuous VADs are less restrictive and allow the patient to practice more activities.

Radial-flow blood pumps were first used in cardio-pulmonary bypass for heart surgery. Based on results obtained with the Bio-Medicus pump (Medtronic Bio-Medicus inc., Eden Prarie, MN), several groups decided to develop much smaller radial-flow blood pumps so that they could be totally implantable. In radial-flow blood pumps, the rotation of the impeller produces a centrifugal force that drags blood from the inlet port on top to the outlet port at the bottom. To produce rotation of the impeller, the impeller is coupled to an electric motor. This coupling is made either (a) magnetically by means of permanent magnets located under the impeller and on the rotor of the motor or (b) mechanically by means of a shaft interposed between the impeller and the motor's rotor. Magnetically coupled devices generally show better functionality because no seal is required between the motor and the impeller shaft.
A problem related to radial-flow blood pumps is that although they are much smaller than pulsatile pumps, they are still too large to be totally implanted in a human thorax thus eliminating any intra-ventricular implantation.
To overcome the above-mentioned problem related to radial-flow blood pumps, axial-flow ventricular assist blood pumps were developed. These axial-flow blood pumps can decrease the hemolysis rate by decreasing the time of exposure of the blood to friction forces and by reducing the intensity of these forces. Another interesting advantage is that axial-flow blood pumps are generally much smaller than radial-flow blood pumps.
The first commercially available axial-flow blood pump was the Hemopump™ (Medtronic Inc. Minneapolis, MN) used as short term circulatory support. Based on the good results obtained with this pump, several groups have initiated the development of totally implantable axial-flow VADs for long term use.
A few axial-flow VADs are presently under intensive development. Examples are: the Jarvik 2000™, by the Texas Heart Institute (Houston, Texas); MicroMed DeBakey™ by MicroMed Technology (also of Houston, Texas); and HeartMate II™, by Thoratec (Pleasanton, California). All of these groups have already started in-vivo experiments on animals and humans although they still perform in-vitro trials. An overview of the operation of these pumps is given hereinbelow.

| **Jarvik 2000™** | This axial-flow blood pump comprises two stators, one at the inflow and one at the outflow. These stators have two functions: they support the bearing shaft around which the impeller will rotate (middle part) and they modify the blood flow path. The inflow stator initiates the rotation of the flow so that the blade tip of the rotor does not create too much shear stress on the blood cells. The outflow stator straightens the flow so that blood from the pump enters the blood stream with a generally axial-flow profile. Permanent magnets are enclosed in the center of the impeller and two motor windings are located in the casing on each side of the rotor. This configuration constitutes a DC brushless motor; this is a simple and durable motor which minimises the number of mechanical parts. The power cable is connected directly to the DC brushless motor controller to change motor speed. |
|---|---|
| | To implant the device, the chest is opened by means of a left thoracotomy and no cardiopulmonary bypass is used. The pump axial outflow is anastomosed to the aorta with a Dacron™ graft. Then a ventriculotomy is made to insert the pump into the ventricle through a sewing ring attached to the apex. |
| **Micromed DeBakey™** | The Micromed DeBakey™ VAD is very similar to the Jarvik 2000™ design. Indeed, this concept of VAD is based on a DC brushless motor with blood-immersed bearings, a central impeller and two fixed side pieces. The Micromed VAD is described in U.S. patent No. 5,527,159 (Bozeman, Jr. et al.) issued on June 18, 1996. |
| | Blood enters on the left side and passes through a flow straightener preventing pre-rotation thereof. Then, the blood reaches the inducer/impetter: the inducer initiates rotation of blood before this blood reaches the impeller. However, it should be noted that the impeller produces the effective pumping action. Finally, a flow diffuser converts the tangential flow into an axial flow. The inducer comprises three blades and the impeller is provided with six blades. The three blades of the inducer co-extend with three associated blades of the impeller. Each blade of the impeller contains eight cylindrical permanent magnets. Finally, a winding is placed outside the pump to complete the motor assembly, and the rotor is supported by a pair of bearings. |
| **HeartMate II™** | This pump is similar to the Micromed DeBakey™ and the Jarvik 2000™ pumps. It is placed next to the heart and is connected between the apex of the heart and the aorta. It is also a sealed-bearing type pump and, accordingly, requires a purge system. This system has a second pump which injects 15 ml/day of sterile solution in the sealed area. A pump without purge system is now under development. Three animals have been supported for one month with the HeartMate II™ (Konishi, H., Antaki, J.F. et al., 1996b, "Long-term Animal Survival with an Implantable Axial Flow Pump as a Left Ventricular Assist Device", *Artificial Organs,* vol. 20, pp. 124-127). |

Other axial-flow blood pumps have been proposed. For example, U.S. patent No. 5,205,721 (Isaacson) issued on April 27, 1993 discloses an axial-flow blood pump having a hydrodynamically suspended rotor centrally positioned with respect to the stator. Hydrodynamic bearings are created by two spaces in which blood must flow to create the hydrodynamic support; this in turn produces shearing forces applied to the blood. In addition, Isaacson teaches three types of impeller blades.

U.S. patent No. 5,211,546 granted to Isaacson et al. on May 18, 1993 teaches an axial-flow blood pump which is similar to that of U.S. patent No. 5,205,721. A rotor is suspended radially by hydrodynamic bearings. In certain embodiments, a radially centered thrust bearing element is provided to stabilise rotation of the suspended rotor.

U.S. patent No. 5,290,227 (Pasque) granted on May 1st, 1994 proposes an axial-flow blood pump having a rotor assembly described generally as a hollowed-out cylinder provided with rotor vanes which extend from the inner surface of the hollowed cylindrical rotor towards the central rotation axis of the rotor. This design generates two pumping zones inside the pump, one of these zones being an outer annulus which is expected to create substantial shearing of the blood in the outer part of the rotor.

European Patent No. EP 0 060 569 granted to Olson et al. on September 22nd, 1982 teaches a magnetically suspended and rotated impeller which comprises a bulky valve member which may be included as part of the impeller. Moreover, this European patent teaches impeller blades which axially extend outside of a shroud to connect to the valve member.

Pumps with impeller blades attached to a hollow cylindrical shaft, the shaft rotating around a fixed axle when a magnetic field is applied, reveal a secondary fluid flow path in the same direction as the primary path. A primary annular flow path is formed between the hollow shaft and the pump housing, and a secondary annular flow is formed between the hollow shaft and the supporting axle. However, these secondary annular paths have minimal flow rates and are used to insure proper lubrication of bearing faces or the cleaning of regions which would otherwise collect debris.

US Patent No. 5 851 174 (Jarvik et al.) describes a cardiac support device, more particularly a cannula pump implantable in the heart, comprising first and second, longitudinally spaced apart pumping blade arrangements for imparting pumping energy to blood entering axially spaced apart first and second inlet ports, respectively, where the blade structure could be modified to provide a mixed flow having both axial and centrifugal components.

## SUMMARY OF THE INVENTION

The present invention relates to a mixed-flow blood pump presenting features of both axial-flow and radial-flow pumps. This mixed-flow blood pump comprises a stationary housing structure and a rotative impeller. The stationary housing structure defines at least one blood inlet, a blood outlet, and a blood conduit between these blood inlet and blood outlet, and the rotative impeller is mounted in the blood conduit. The rotative impeller has an impeller shaft rotative about the longitudinal axis of the stationary housing structure which has a shaft portion tapered in a direction opposite to the direction of blood flow. The at least one blood inlet, the blood outlet, the blood conduit and the rotative impeller have respective structures and configurations that operate the mixed-flow blood pump at a given point of a maximum hydraulic efficiency curve relating a specific pump rotational speed and a specific pump diameter, that given point being located within a transition region of the maximum hydraulic efficiency curve between axial-flow and radial-flow pumps.

Operating a mixed-flow blood pump, which presents the flow characteristics of an axial-flow blood pump while maintaining the throughput of a radial-flow blood pump, at the given point of the maximum hydraulic efficiency curve located within the transition region of the curve between radial-flow and axial-flow pumps enables obtention of a pumping efficiency as high as 53%.

The foregoing and other objects, advantages and features of the present invention will become more apparent upon reading of the following non-restrictive description of illustrative embodiments thereof, given by way of example only with reference to the accompanying drawings.

## BRIEF DESCRIPTION OF THE DRAWINGS

In the appended drawings:

Figure 1 is a cross sectional view of a human heart in which an illustrative intra-ventricular embodiment of the mixed-flow blood pump according to the present invention is installed;

Figure 2 is a graph showing, for different types of pumps, a curve relating a specific pump rotation speed $N_s$ with a specific pump diameter $D_s$ at the points where the pump is operating at maximum hydaulic efficiency;

Figure 3 is a side elevational view of the external shape of the illustrative intra-ventricular embodiment of the mixed-flow blood pump according to the present invention;

Figure 4a is a side elevational and cross sectional view of the illustrative intra-ventricular embodiment of the mixed-flow blood pump according to the present invention;

Figure 4b is a side elevational and cross sectional view of an illustrative extra-ventricular embodiment of the mixed-flow blood pump according to the present invention;

Figure 5a is an enlarged, partial side cross sectional view showing a first end mount of the drive shaft of the mixed-flow blood pump of Figure 4a or 4b;

Figure 5b is an enlarged, partial side cross sectional view showing a second end mount of the drive shaft of the mixed-flow blood pump of Figure 4a or 4b; and

Figure 6 is a schematic view of an illustrative embodiment of a VAD system implanted in a human being and comprising the mixed-flow blood pump of Figure 4a.

## DESCRIPTION OF THE ILLUSTRATIVE EMBODIMENT

In the following description, important aspects of the pump design are addressed. In particular, the pump design takes into consideration anatomical and physiological considerations combined with mechanical, electrical and material requirements. Finally, following this discussion, global characteristics of a VAD system are presented.

It should first be noted that the mixed-flow blood pump of the present invention is not restricted to an application to an implantable VAD system. Since the mixed-flow blood pump according to the invention overcomes a number of drawbacks of the current blood pumps, those of ordinary skill in the art will understand that such a mixed-flow blood pump can be used as part of an intra-corporal system such as an intra-ventricular VAD, or an extra-ventricular VAD (for example a VAD located in the abdomen or thorax), or alternatively as a para-corporal or extra-corporal VAD (for example in a bridge to heart transplantation). It shall also be understood that the mixed-flow blood pump of the present invention can be used in temporary VADs (for example a bridge to heart transplant) or permanent VADs:

## ANATOMICAL, PHYSIOLOGICAL AND SURGICAL CONSIDERATIONS

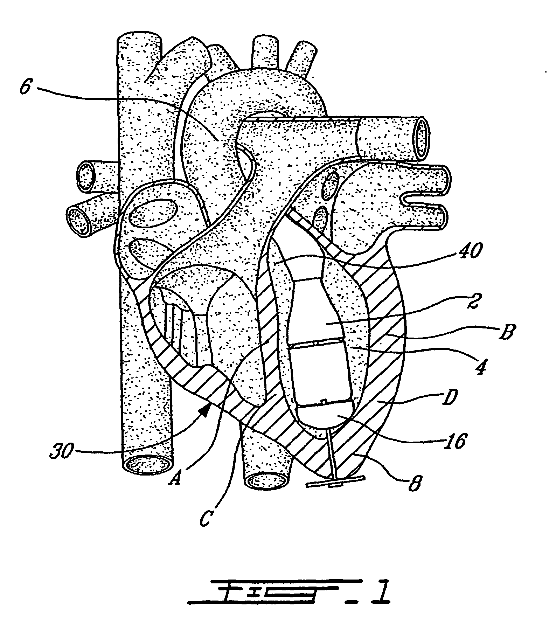

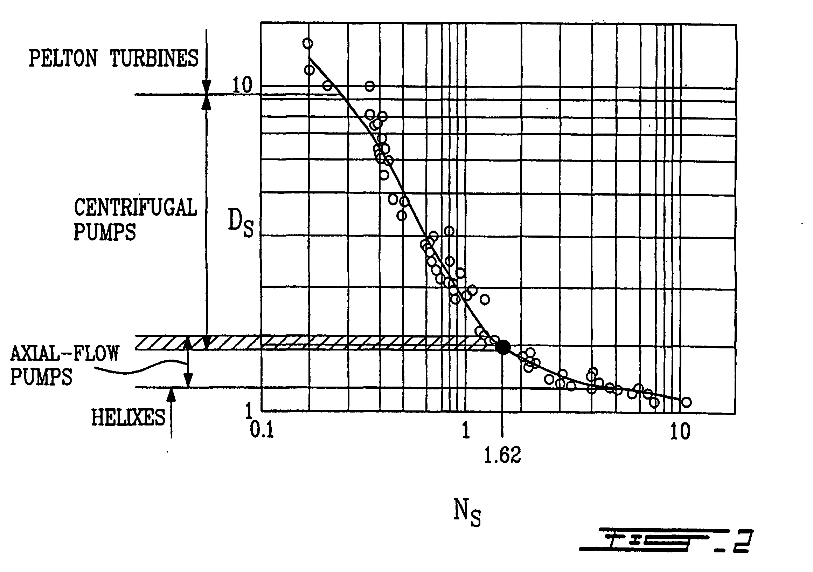

As previously discussed, bleeding is an important problem associated with patients who receive a VAD; in fact 30 % of patients suffer from this problem (Defraigne, J.O., Limet, R., 1996a, "Les assistances circulatoires: Partie I. Indications et description des systèmes", *Rev Med Liege,* Vol. 51, pp.295-306). The risk of infection is another quite important problem. These medical and surgical considerations are met by the illustrative intra-ventricular embodiment of the mixed-flow blood pump as shown in Figure 1. This position eliminates the need for inflow and outflow grafts and their anastomoses to thereby reduce the risk of bleeding and infection. This has also the obvious advantage of considerably reducing the implantation time. Figure 1 illustrates the proposed position of the illustrative intra-ventricular embodiment of mixed-flow blood pump 2 in the left ventricle 4.

The mixed-flow blood pump 2 has also been designed to fit in small adults and in teens. Since the physical size and shape of the mixed-flow blood pump 2 are greatly influenced by the desired location of the pump, a good description of the ventricle anatomy is required. Feigenbaum, Harvey, "Echocardiography", 5th edition, 1994, Lea & Febiger, Philadelphia, presents several dimensions of the heart normalised by the BSA (Body Surface Area). These anatomical dimensions have been statistically determined and are known to represent 95% of the population. Taking into consideration the above-identfied statistics, a ventricular dimension for humans corresponding to a BSA of 1.5 $m^2$ was used to design the pump.

It will be understood that the physical size and shape of the mixed-flow blood pump 2 could also be adapted to meet the anatomical dimensions of individuals falling outside this 95% of the population. Similarly, the size and shape could be adapted to specific and particular individuals and heart conditions.

For 95% of the population, the internal diameter of the left ventricle 4 ranges from 37 to 46 mm in diastole and between 22 to 31 mm in systole. This diameter is determined at the centre of the ventricular length (segment AB in Figure 1). The diameter near the apex at the first third of the ventricular length is about 1.5 cm (segment CD of Figure 1). The internal length of the ventricle from the apex to the aortic valve ranges from 55 to 70 mm. Finally, the other important parameter is the surface of the aortic valve opening which ranges from 2.5 to 4 $cm^2$.

From a surgical point of view, the favoured insertion procedure is to use the same approach as with valve replacement. According to this procedure, an incision is made at the root of the aorta 6 (Figure 1) and the mixed-flow blood pump 2 is inserted though the aortic valve and then into the left ventricle 4. The mixed-flow blood pump 2 is then pushed until its base reaches the myocardium at the apex 8. In order to prevent motion thereof, the mixed-flow blood pump 2 should be fixed. Also in accordance with an illustrative embodiment, an outflow cannula should pass through the aortic valve to further reduce bleeding.

One of the main roles of the mixed-flow blood pump 2 is to restore a hemodynamic function in patients with cardiac failure. Depending on the severity of the failure and the BSA, the pump 2 is susceptible to work at flow rates between 2 to 6 litres per minute (l/min) against a pressure as high as 120 mmHg and, more commonly, at a flow rate between 3 to 5 l/min against a pressure of 80 mmHg.

Another important consideration for blood pump design is the hemolysis rate. Hemolysis is the tearing of red blood cells which empties the content of the cells in the blood stream resulting in free haemoglobin; the normal level of plasma free haemoglobin is around 10 mg/dl. A blood pump with a normalised index of hemolysis (NIH) of 0.005 g/100 l and lower is considered to be almost athromatic for red blood cells. A NIH of about 0.05.g/100 l could be tolerated.

A NIH situated between 0.005 g/100 l to 0.05 g/100 l can therefore be envisaged for a VAD. Of course, a NIH as close to 0.005 g/100 l as possible is desirable.

Platelets are other important blood elements; their activation by high hydromechanical forces should be avoided in order to prevent clot formation.

MIXED-FLOW FLOW BLOOD PUMP DESIGN: Mechanical aspects

This section of the disclosure is divided into parts A, B and C. Part A describes the general approach used for the selection of the pump configuration. Part B describes the external shape and size of an illustrative embodiment of the mixed-flow blood pump according to the invention, and part C describes the internal structure of this illustrative embodiment of mixed-flow blood pump.

**Part A:** Selection of a general pump configuration

There are three existing non-pulsatile pump configurations, all turbines, and having characteristics which make them potential candidates for a cardiac blood pump: radial-flow, axial-flow and mixed-flow pumps. Given their relatively small diameter, cylindrical shape and high throughput, axial-flow pumps display a number of characteristics which make them particularly well suited for implantation. However, other pump configurations also exhibit characteristics, different from those of the axial-flow pump, which would also be useful in a cardiac blood pump.

When designing turbine pumps, dimensionless characteristic values are used to compare different pump configurations. Dimensionless characteristic values provide useful indications to pump designers of expected performance regardless of the size of the pump, a comparison which would otherwise prove difficult given a virtually infinite number of operating parameters that depend on infinite variations of internal pump geometry. These dimensionless characteristic values, therefore, can be used to provide an objective starting point for the selection of a general pump configuration.

Two of these dimensionless characteristic values are the specific rotation speed $N_s$ of the pump and the specific pump diameter $D_s$. They are defined as follows:

$$N_s = \frac{\Omega \; Q^{1/2}}{H^{3/4}} \qquad\qquad (1)$$

$$D_s = \frac{D \cdot H^{1/4}}{Q^{1/2}} \qquad\qquad (2)$$

where $\Omega$ is the rotation speed the pump in rad/s, Q is the flow rate in m³/second, H is the head (i.e. the gain in pressure) of the pump and D the diameter of the pump, both in meters. $N_s$ remains the same regardless of the size of the pump and therefore provides an accurate measure of the performance of a given pump design. $D_s$ relates the pump diameter to the pump head H and flow rate Q.

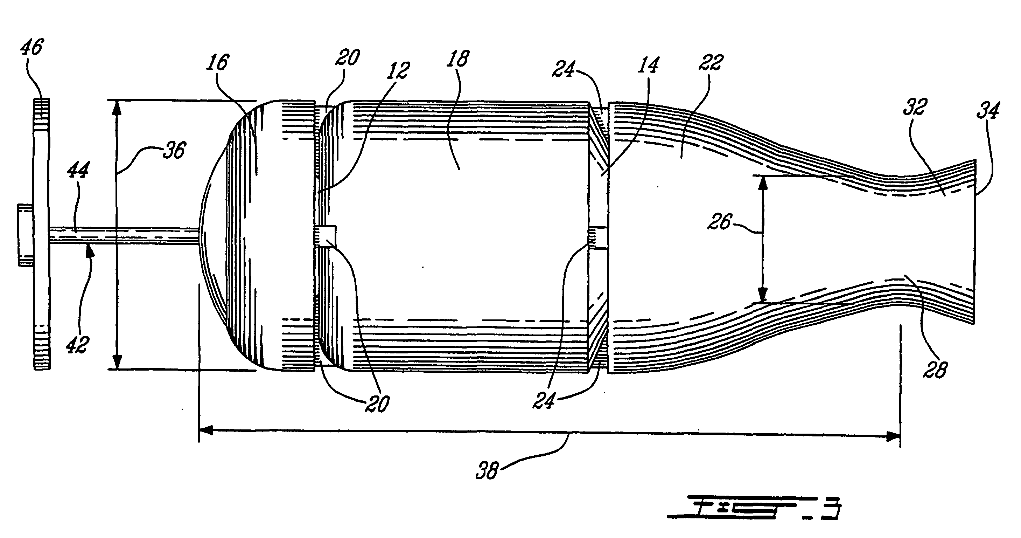

Referring to Figure 2, known are curves which relate the specific speed $N_s$ with the specific diameter $D_s$ at the points where the pump is operating at maximum hydraulic efficiency. In this regard, hydraulic efficiency is expressed as the percentage of the power input to the pump which is converted to energy of movement of the fluid within the pump. From the curve of Figure 2 and equation (1) above, it follows that optimally efficient pumps having a higher specific speed also have a smaller size.

As referred to above, there are three (3) principal categories of non-pulsatile pumps characterised by the direction of flow of fluid through the pump relative to the axis of rotation: axial-flow, radial-flow and mixed-flow pumps.

In axial-flow pumps the direction of fluid flow is parallel to the axis of rotation. The pressure differential, or head, is produced by a change in the amount of tangential movement. Characteristics associated with axial-flow pumps include high flow rate Q and small head H. This results in high specific speeds $N_s$.

In radial-flow pumps a large portion of the throughput, either on the outlet or the inlet, is radial, i.e. perpendicular to the axis of rotation. This change in direction causes an increase in pressure. Contrary to an axial-flow pump, a radial-flow pump is characterised by relatively large head H and smaller flow rate Q, resulting in lower specific speeds $N_s$.

Located between radial-flow pumps and axial-flow pumps are mixed-flow pumps where the direction of flow at the output or input is composed of both radial-flow and axial-flow components. As would be expected, the specific speed of mixed-flow pumps is located between the specific speed of axial-flow and radial-flow pumps.

In order to determine an optimised choice for a pump, it is necessary to evaluate the specific speed $N_s$ in light of the characteristics in terms of head H and flow rate Q projected for the pump. As discussed above, the pump will typically be operated with a flow rate of 5 l/min and a head of approximately 100 mmHg. Additionally, current motor technology provides small yet efficient motors operating at a speed of 11,000 RPM. This gives a specific speed of 1.62.

Referring again to Figure 2, an indication is given to the ranges of $N_s$ and $D_s$ within which a given pump configuration will provide efficient operation. The specific speed $N_s$ of 1.62 falls within a transition region of the curve between axial-flow and radial-flow pumps. In this transition region, a mixed-flow pump topology would yield a higher efficiency than purely radial-flow or axial-flow pumps. Additionally, the specific diameter $D_s$ is around 2 which, by applying equation (2) above, yields a characteristic diameter of $9.83 \times 10^{-3}$ for the pump, i.e. a very small pump.

**Part B:** External design requirements

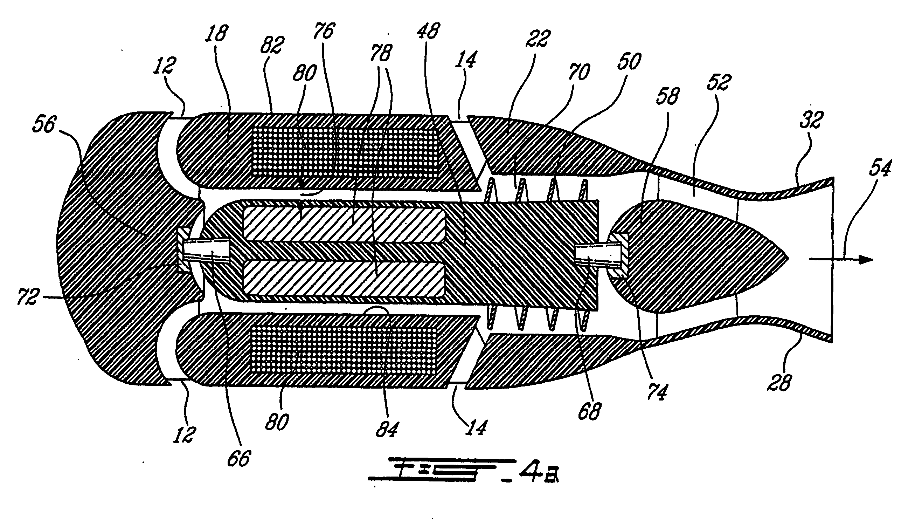

The external design (shape and size) of the mixed-flow blood pump 2 (Figure 1) depends on the anatomic dimensions of the left ventricle 4. Figure 3 illustrates the external shape of the mixed-flow blood pump 2 and the critical geometric parameters thereof.

**Pump Inlets**

Figure 1 shows that the illustrative embodiment of the mixed-flow blood pump 2 rests at the bottom of the left ventricle 4, in the region of the apex 8 of the heart 30.

Referring to Figure 3, in order to prevent the inner walls of the left ventricle 4 from completely obstructing blood intake, two axially spaced apart inlets 12 and 14 are provided. Additionally, a first end of the pump presents the generaly configuration of a hemisphere 16. The diameter of the hemisphere 16 is set to approximately 20 mm, which is smaller than the segment CD (see Figure 1) and suitable to limit the level of pressure on the walls of the left ventricle 4 near the apex 8.

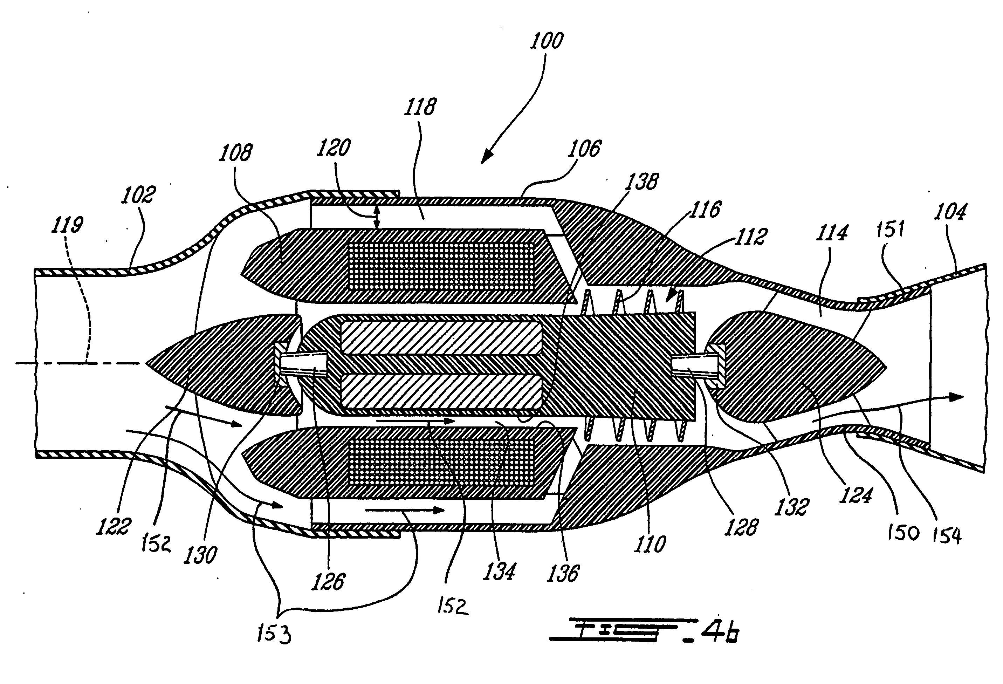

The inlet 12 is found at the first end of the pump 2 between the hemisphere 16 and a first end of an axial, cylindrical member 18. As illustrated in Figure 4, the cylindrical member 18 contains the stator windings 80. A first series of narrow, axially extending supports 20 spread out evenly around the axis of the pump 2 connects the hemisphere 16 to the cylindrical member 18.

The second inlet 14 is formed between the second end of the cylindrical member 18 and an impeller housing 22. A second series of narrow, axially extending supports 24 spread out evenly around the axis of the pump 2 interconnects the cylindrical member 18 to the impeller housing 22. As can be seen, the second inlet 14 is axially spaced apart from the first inlet 12. The separation between the inlets 12 and 14 reduces the effect occlusion of one of the pump inlets may have on normal operation of the pump 2.

**Outflow Cannula**

Referring back to Figures 1 and 3, at the second end of the mixed-flow blood pump 2, the outflow diameter 26 (see Figure 3) is reduced so as to reduce as much as possible the obstruction caused by an outflow cannula 28 to the operation of the aortic valve (not shown); since the function of the mixed-flow blood pump 2 is to assist blood circulation, blood flow contribution from the natural contraction of the heart 30 should be maintained. In an illustrative embodiment, the area of the outflow cannula 28, corresponding to diameter 26, is 1.3 cm$^2$.

As illustrated, the outflow cannula 28 is, in the illustrative embodiment, integral with the impeller housing 22.

As can be better seen from Figure 3, a blood diffuser 32 is formed at the free end of the cannula 28, integrally therewith. The function of the diffuser 32 is to reduce the shear stress on blood cells. Without diffuser 32, the velocity of blood ejected from the pump 2 is higher than the velocity of blood ejected from the heart 30. The difference in velocity between these two blood flows would result in shear stress proportional to this difference. Since the velocity is inversely proportional to the cross-sectional area, a solution for reducing the relative velocity of the blood flows from the pump 2 and from the heart 30 is (a) to increase the area of the orifice 34 of the cannula 28 to reduce the velocity of the flow of blood from the pump 2, and (b) to decrease the area occupied by the blood flow from the heart to increase the velocity of the latter blood flow. This is exactly the role of the diffuser 32. Of course, parameters such as the angle of opening and the length of the diffuser 32 can be adjusted at will to fit the mechanical characteristics of the pump 2 in view of minimising the shear stress on the blood cells.

**Housing**

The diameter of the mixed-flow blood pump 2 is a compromise between pumping requirements and minimal interference with heart contraction. In an illustrative embodiment, the maximum allowable diameter 36 is about 22 mm which is the diameter of the left ventricle 4 in systole. This dimension is reasonable since people with heart failure generally have dilated ventricles.

The maximum length 38 of the mixed-flow blood pump 2, as illustrated in Figure 2, is set in regard of the average distance between the apex 8 and the aortic valve 40 of the heart 30. In an illustrative embodiment, the length 38 of the mixed-flow blood pump 2 is about 55 mm. As shown in Figure 1, a reduction of the pump diameter (see 40) toward the outflow increases the aortic valve clearance in order to minimise interference with the aortic valve function.

Since in this illustrative embodiment the mixed-flow blood pump 2 will be completely located inside the left ventricle 4, blood will circulate around the pump 2. As a consequence, all external surfaces of the pump 2 should be as smooth as possible and avoid as much as possible abrupt deviations to thereby minimise vortices, turbulence and recirculation zones which may be at the origin of clot formation. To overcome this problem, the pump 2 and other components may be machined from surgical quality titanium.

**Fixation Mechanism**

At the first end of the pump 2, a fixation mechanism 42 is provided. As an example, fixation mechanism 42 comprises:

- an elongated needle member 44 extending from the hemisphere 16, this needle member 44 being driven from the inside of the left ventricle 4 through the myocardium and the epicardium at the apex 8 of the heart 30; and

- a fixation disk 46 fastened to the free end of the needle member 44 on the outside of the heart 30 to firmly fix the mixed-flow blood pump 2 within the left ventricle 4. Of course, it is within the scope of the present invention to employ any other type of fixation mechanism.

**Electrical Supply**

The required electrical supply for the operation of the motor (to be described herein below) is made through a wire that could, for example, extend from the mixed-flow blood pump 2 along the needle member 44 to reach a controller and an energy source (both to be further described hereinafter).

**Part C**: Internal design characteristics

In the design of the internal components, some of the characteristics of axial-flow pumps have been retained and combined with some of the characteristics of radial-flow pumps to form the mixed-flow blood pump 2.
Referring to Figure 4a, the mixed-flow blood pump 2 comprises a stationary housing structure comprising an inflow bushing mount 56 which defines the hemisphere 16, the cylindrical member 18, the impeller housing 22, a stationary outflow stator 52, an outflow bushing mount 58, the outflow cannula 28 and the blood diffuser 32. Blood pump 2 further comprises a rotative impeller 50 with an impeller shaft 48 and an impeller blade 70.
Current wet motor axial-flow blood pumps use permanent magnets inserted either in the central hub or in the impeller blades. Both methods require important compromises. Insertion of the permanent magnets in the central hub requires a large hub to locate the permanent magnets close to the motor windings for obvious electromagnetic coupling reasons. In contrast, a small hub has the advantage of increasing the pumped volume of blood. Insertion of the permanent magnets in the impeller blades yields a compromise since the geometry of the blades must be curved for pumping efficiency. As a consequence, some of the embedded magnets move away from the windings as the blade (s) curve(s).
In the approach proposed by the illustrative embodiment of the present invention, the mixed-flow blood pump 2 presents an enclosed-impeller mixed-flow configuration. In the illustrative intra-ventricular embodiment of this configuration as illustrated in Figure 4a, the impeller blade 70 is a spiralling auger-type impeller blade having a constant height and being rigidly attached to the outer surface of the impeller drive shaft 48 and enclosed in the impeller housing 22. Obviously, the impeller blade 70 fits snugly within the impeller housing 22. Of course, the shape (curvature and angulation) of the impeller blade 70 should be optimally designed in relation to pumping performance and other hydrodynamic considerations. In particular, the influence of the blade angulation on the level of shearing stresses, turbulence and cavitation responsible for red blood cell damage and increase of hemolysis rate must be carefully taken into consideration.
The end portion of the impeller shaft 48 bearing the impeller blade 70 is slightly tapered in a direction opposite to the direction of blood flow. This contributes to create the mixed-flow operation of the pump 2. More specifically, this slight taper imparts to the blood flow both axial and radial components.
Still referring to Figure 4a, at both ends of the impeller drive shaft 48 end pivots 66 and 68 protrude. The function of the end pivots 66 and 68 is to support the impeller drive shaft 48 at each end. The end pivots 66 and 68 are respectively inserted into the respective bushing mounts 56 and 58. Bushing 72 is mounted on the inner face of the inflow bushing mount 56. In the same manner, bushing 74 is mounted on the adjacent face of the outflow bushing mount 58. The bearings formed by the bushing and pivot assemblies 66;72 and 68;74 are the only mechanical parts subject to wear. Therefore, these parts are expected to be mostly responsible for the life span of the mixed-flow blood pump 2.

Still referring to Figure 4a, the cylindrical gap 76 separating the impeller shaft 48 and the inner surface of the cylindrical member 18 should be sufficiently thick to produce sufficient blood flow in order to increase washout and prevent clot formation. On the other hand, too large a gap 76 may either reduce the pump efficiency (by reducing the electromagnetic coupling) or result in higher hemolysis. Just a word to mention that the section of the impeller shaft 48 within the cylindrical member 18 is cylindrical whereby the gap 76 has a constant thickness.

In the illustrative embodiment of Figures 1 and 4a, the volume of blood pumped through the second inlet 14 is typically 4 liters/minute. This is higher than the volume of blood pumped through the first inlet 12 and the cylindrical gap 76 which is typically 1 liter/minute. A number of benefits is associated with the higher volume blood pumped through the second inlet 14. For example, installation of the mixed-flow blood pump 2 in the left ventricle 4 of a patient with the cannula 28 extending through the aortic valve generally interferes with proper operation of the aortic valve. Optimally, the aortic valve should continue to function normally; however, in some cases, it has been observed that the aortic valve ceases to function further until it remains closed around the cannula 28. Typically, blood would have the tendency to collect in the region close to the aortic valve and the cannula 28 which might lead to thrombus formation and other adverse effects. The increased volume of blood pumped through the second inlet 14 has the effect of creating turbulence in the region within the ventricle 4 bordered by the aortic valve and the cannula 28, thus providing improved washout of this region and thereby reducing the effects of the malfunctioning aortic valve.

On the one hand, the volume of blood pumped through the second inlet 14 contributes to the radial-flow operation of the mixed-flow blood pump 2. On the other hand, the volume of blood pumped through the first inlet 12 and the cylindrical gap 76 contributes to the axial-flow operation of the mixed-flow blood pump 2.

The stationary outflow stator 52 comprises a plurality of blades shaped and disposed around the outflow bushing mount 58 to transform the rotational motion of the flow about the longitudinal axis 54 into a translational motion. Therefore, the stationary outflow stator 52 constitutes a blood flow straightener.

As previously mentioned, the pump design should minimise shearing stress in order to minimise hemolysis. In that context, reduction of the rotational speed would obviously contribute to reduce hemolysis. However, reduction of the rotational speed while pumping the same volume of blood requires an increase of the volume of blood contained in the rotor zone of the pump 2. The volume of blood contained in the rotor zone can be increased by either increasing the diameter of the rotor zone, or alternatively minimising the volume of the central hub of the pump rotor.

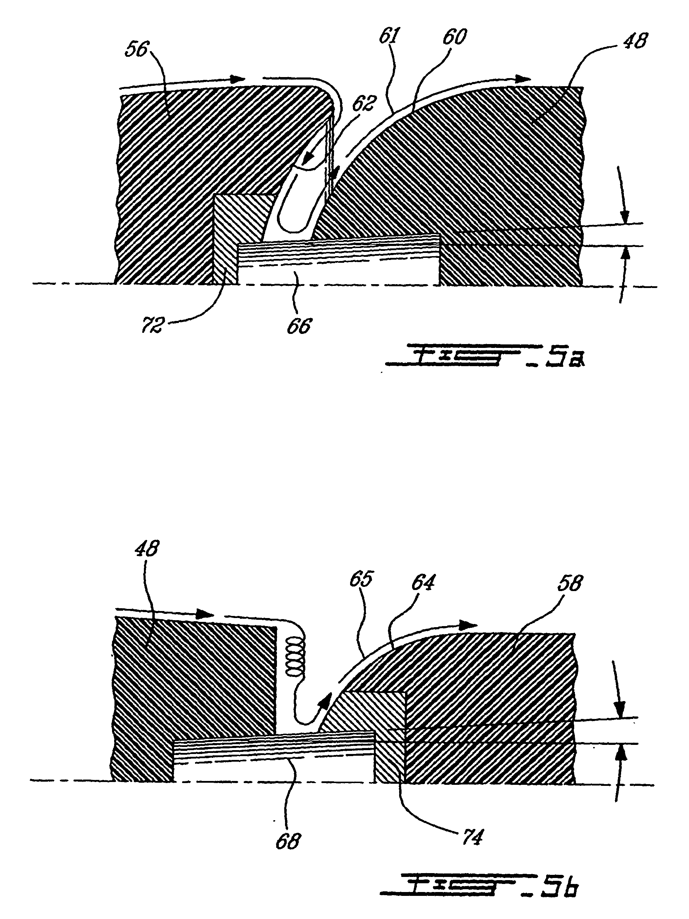

Design of the internal surfaces of the pump is also important for minimising shearing stresses in order to minimise hemolysis. Figure 5a shows the configuration of the region between the inflow bushing mount 56 and the impeller drive shaft 48. Figure 5b shows the configuration of the region between the impeller drive shaft 48 and the outflow bushing mount 58.

Referring to Figure 5a, the end surface 60 of the impeller drive shaft 48 is convex and generally hemispheric while the confronting surface 62 of the bushing mount 56 is concave and generally hemispheric. Forming the surfaces in this manner reduces the shearing stress placed on the blood thus minimising hemolysis (see flow 61).

The outflow bushing mount 58 is formed with a similar convex, generally hemispheric end surface to reduce shearing stress at the outlet (see Figure 5b, in particular flow 65). The confronting end of the impeller shaft 48 is flat and perpendicular to the axis 54.

Additionally, referring to both Figures 5a and 5b, end pivots 66 and 68 by which the respective ends of the impeller shaft 48 are mounted to their respective bushing mounts 56, 58 are slightly tapered in a direction opposite to blood flow. This helps prevent the creation of eddies and the collection of debris in proximity to the end pivots. Normally, a taper of the order of five degrees (5°) is adequate.

In this manner, pivot 66 has a smaller diameter free end received within the bushing 72. In the same manner, the pivot 68 has a larger diameter free end received in the bushing 74.

Figure 4b illustrates an alternative, illustrative extra-ventricular embodiment of mixed-flow blood pump 100. Pump 100 is adapted for use externally of the heart as a ventricle bypass/assist. In this embodiment the pump 100 would typically be implanted above the diaphragm in the thorax and would be connected to the circulation system using standard vascular grafts, a first graft 102 being attached to the inflow end of the pump and a second graft 104 being attached to the outflow end of the pump 100.

Similar to the mixed-flow blood pump 2, the attemative illustrative embodiment 100 as illustrated in Figure 4b comprises a stationary housing structure including an inflow bushing mount 122, a cylindrical member 108, an impeller housing 106, a stationary outflow stator 114, an outflow bushing mount 124, an outflow cannula 150 and a blood diffuser 151. Blood pump 100 further comprises a rotative impeller 112 with an impeller shaft 110 and an impeller blade 116.

The impeller blade 116 is a spiralling auger-type impeller blade rigidly connected to the impeller drive shaft 110 and enclosed in the impeller housing 106.

A series of longitudinal ridges 118, typically five (5) evenly spaced around the pump axis 119, support the cylindrical member 108 within the impeller housing 106 thereby forming a series of longitudinal flow passages such as 120 between the cylindrical member 108 and the impeller housing 106.

The longitudinal ridges 118 extend to meet and hold rigid the inflow bushing mount 122 with respect to the impeller housing 106 and the cylindrical member 108. Similarly, the outflow bushing mount 124 is supported within the impeller housing 106 through the stationary outflow stator 114.

The stationary outflow stator 114 comprises a plurality of blades shaped and disposed around the outflow bushing mount 124 to transform the rotational motion of the blood flow about the longitudinal axis 119 into a translational motion. Therefore, the stationary outflow stator 114 constitutes a flow straightener.

At both ends of the impeller shaft 110 end pivots 126 and 128 protrude. The end pivots 126 and 128 are respectively inserted into respective bushings 130 and 132 to support the impeller drive shaft 110 within the cylindrical conduit 108 while at the same time allowing the impeller shaft 110 to rotate freely. Bushing 130 is mounted on the inflow bushing mount 122. In the same manner, bushing 132 is mounted on the outflow bushing mount 124.

In addition to the series of longitudinal flow passages 120, an annular flow passage 134 is formed between the inner surface 136 of the cylindrical member 108 and the outer surface 138 of the impeller shaft 110.

Flow of blood through the mixed-flow pump 100 is indicated by the arrows 152-154.

The other characteristics of the mixed-flow pump 2 according to the illustrative embodiment of Figure 4a also apply to the illustrative embodiment 100 of Figure 4b.

**Electrical Aspects**

In the illustrative embodiment of Figure 4a, the mixed-flow blood pump 2 is actuated by means of a brushless DC (direct current) motor. This brushless configuration presents the advantage of minimal wear. Two other interesting characteristics of brushless DC motors are high rotational speed and high torque.

In the mixed-flow blood pump 2, the brushless DC motor includes elongated axial permanent magnets such as 78 inserted in the impeller shaft 48 and stator windings 80 embedded or housed in the cylindrical member 18.

As discussed previously, the cylindrical gap 76 between the outer surface of the impeller shaft 48 and the inner surface of the cylindrical member 18 must be sufficiently thick to produce sufficient blood flow in order to increase washout and prevent clot formation. However, increasing the thickness of the gap 76 decreases the efficiency of the magnetic coupling between the permanent magnets 78 and the stator windings 80. This requires an increase in current through the stator windings 80 to compensate for the decreased efficiency and to maintain the same characteristics in terms of impeller blade speed and blood volume throughput. Of course, increase in current leads to an increase in thermal loss from the stator windings 80; this thermal loss increases as the square of the current through the stator windings 80. As the temperature of the surface of the stator windings must remain at or below 40°C, the gap 76 must be sufficiently small to provide efficient magnetic coupling between the permanent magnets 78 and the stator windings 80.

Thermal performance is also improved given the proximate position of the stator windings 80 to the external surface 82 of the cylindrical member 18. Blood flow over the external surface 82 efficiently cools the stator windings 80. The flow of blood within the gap 76 between the impeller shaft 48 and the inner surface 84 of the cylindrical member 18 also contributes in efficiently cooling the stator windings 80.

The alternative illustrative embodiment 100 of the mixed-flow blood pump as illustrated in Figure 4b maintains the essential electrical characteristics of the illustrative embodiment of Figure 4a with the exception that, referring to Figure 4b, the design of the pump 100 overcomes the thermal limitations by allowing for a second blood flow passage along the series of longitudinal flow passages 120 between the impeller housing 106 and the cylindrical member 108.

Axial spacing between the impeller blade and the permanent magnets along the impeller shaft enables separate design of the DC motor and the impeller to obtain simultaneously both efficient coupling between the permanent magnets and the stator windings and sufficient pumping volume.

**Selection of the Materials**

The choice of materials for an implantable device is crucial and several properties of the available materials should be considered: strength, durability, hardness, elasticity, wear resistance, surface finish and biocompatibility. Biocompatibility is very important to minimise irritation, rejection and thrombogenesis. The interaction between the surface of the material and the biological tissues is very complex. In several cases, treatment of the surface with human proteins, certain drugs like heparin or other biocompatible material may considerably increase the biocompatibility and minimise thrombus formation.

**VAD SYSTEM**

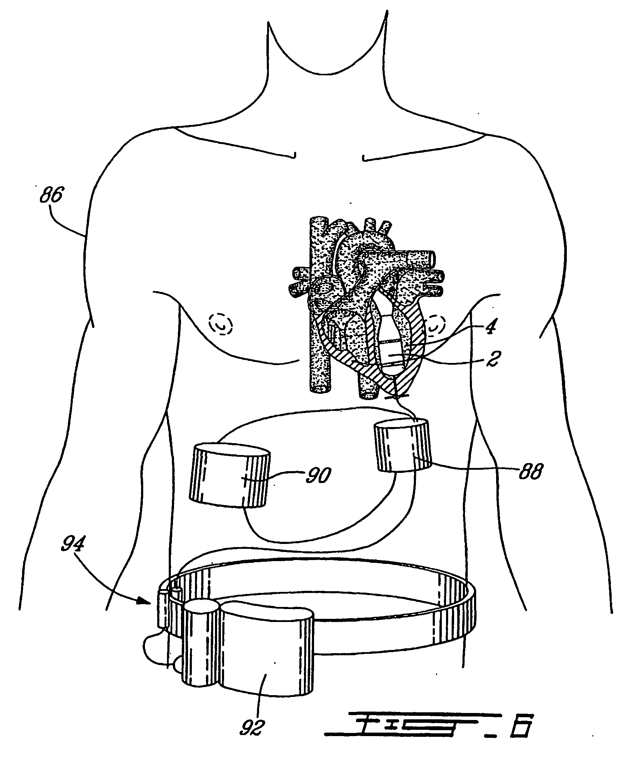

Figure 6 schematically illustrates an embodiment of implantable VAD system including an axial-flow blood pump 2. The VAD system is composed of four main parts:

- the axial-flow blood pump 2 implanted in the left ventricle 4 of the patient 86;

- an internal controller 88;

- two energy sources, namely an internal rechargeable battery 90 and an external rechargeable battery 92; and

- a Transcutaneous Energy and Information Transmission (TEIT) system 94.

VAD and TEIT Systems are well known in the art and will not be further discussed in the present specification.

To conclude, ventricular assist devices (VADs) are now being used worldwide and their utilisation is becoming more and more accepted as a solution to treat end stage heart failure. It is generally accepted that VADs extend life of patients while improving quality of life of these patients. A poll, made with patients who received VADs, concerning their quality of life revealed that these patients would have preferred a heart transplant but prefer their situation than having to be on dialyses.

It is also now being accepted that VAD is becoming a cost effective solution considering the fact that patients are discharged from the hospitals more rapidly and may return to normal life occupations. In the United States, several insurance companies are now reimbursing the implantation of VADs.

Finally, the mixed-flow blood pump 2 according to the invention provides an excellent bridge to heart transplant and aims at long term implant. The new proposed mixed-flow blood pump 2 should answer most of the remaining problems and limitations of the prior axial-flow blood pumps, especially those related to hemolysis and bleeding.

Although the present invention has been described hereinabove by way of illustrative embodiments thereof, these embodiments can be modified at will, within the scope of the appended claims.

## Claims

1. A mixed-flow blood pump (2) presenting features of both axial-flow and radial-flow pumps, comprising:

   a stationary housing structure (22) defining a longitudinal axis (54), an axially-extending annular blood inlet passage (76) a radially-extending annular blood inlet passage (14) an axial blood outlet (28), and an axial blood conduit between (a) the axially-extending and radially-extending blood inlet passages, and (b) the axial blood outlet; and
   an rotative impeller (50) mounted within the stationary housing structure, comprising:

      an impeller shaft (48) rotative about the longitudinal axis of the stationary housing structure, the impeller shaft having, in the axial blood conduit, a shaft portion tapered in a direction opposite to the direction of blood flow; and
      an impeller blade (70) mounted on the tapered shaft portion;

   - wherein the axially-exteriding annular blood inlet passage, the radially-extending annular blood inlet passage, the tapered shaft portion, the impeller blade mounted on the tapered shaft portion, and the axial blood outlet can operate the mixed-flow blood pump at a given point of a maximum hydraulic efficiency curve relating to a specific pump rotational speed and a specific pump diameter, said given point being located within a transition region of the maximum hydraulic efficiency curve between axial-flow and radial-flow pumps.

2. A mixed-flow blood pump as defined in claim 1, wherein the specific pump rotation speed and the specific pump diameter have dimensionless values.

3. A mixed-flow blood pump as defined in claim 2, wherein the dimensionless values of the specific pump rotational speed is 1.62.

4. A mixed-flow blood pump as defined in claim 1, wherein:

   the stationary housing structure comprises first (12) and second (14) axially spaced apart annular blood inlets;
   the axially-extending annular blood inlet passage extends between the first annular blood inlet and the axial blood conduit; and
   the radially-extending annular blood inlet passage extends between the second annular blood inlet and the axial blood conduit.

**5.** A mixed-flow blood pump as defined in claim 4, wherein:

the axially-extending annular blood inlet passage comprises a generally cylindrical axial passage portion.

**6.** A mixed-flow blood pump as defined in claim 4, wherein:

the radially-extending annular blood inlet passage defines an acute angle with respect to the longitudinal axis of the stationary housing.

**7.** A mixed-flow blood pump as defined in claim 5, wherein:

the stationary housing structure comprises a cylindrical member with an inner surface; and
the generally cylindrical, axial passage portion comprises a gap between the impeller shaft and the inner surface of the cylindrical member.

**8.** A mixed-flow blood pump as defined in claim 1, in which the impeller blade comprise an auger-type anpeller blade mounted on the impeller shaft.

**9.** A mixed-flow blood pump as defined in claim 1, wherein:

the stationary housing structure comprises a cylindrical member around the impeller shaft; and
the mixed-flow blood pump further comprises an electrical motor structure comprising:

- permanent magnets embedded within the impeller shaft; and
- electrical windings mounted within the cylindrical member of the stationary housing structure.

**10.** A mixed-flow blood pump as defined in claim 7, wherein;
the mixed-flow blood pump further comprises, in the region of the gap between the impeller shaft and the inner surface of the cylindrical member, an electrical motor structure comprising:

- permanent magnets embedded within the impeller shaft; and electrical windings mounted within the cylindrical member of the stationary housing structure.

**11.** A mixed-flow blood pump as defined in claim 1, wherein:

the impeller shaft comprises first and second opposite ends and first and second opposite end pivots; and
the stationary housing structure comprises first and second bushing mounts at the respective first and second ends of the impeller shaft to receive the first and second opposite end pivots, respectively.

**12.** A mixed-flow blood pump as defined in claim 11, wherein:

the first pivot is slightly tapered in a direction opposite to the direction of blood flow and comprises a smaller diameter free end;
the first bushing mount comprises a first bushing to receive the smaller diameter free end of the first pivot;
the first bushing mount comprises a concave, generally hemispheric surface in the region of the first bushing; and
the first end of the impeller shaft is convex and generally hemispheric.

**13.** A mixed-flow blood pump as defined in claim 11, wherein:

the second pivot is slightly tapered in a direction opposite to the direction of blood flow and comprises a larger diameter free end;
the second bushing mount comprises a second bushing to receive the larger diameter free end of the second pivot;
the second bushing mount comprises a convex, generally hemispheric surface in the region of the second bushing; and
the second end of the impeller shaft is generally flat.

**14.** A mixed-flow blood pump as defined in claim 1, wherein:

the stationary housing structure comprises an impeller housing around the impeller blade, the impeller housing having an inner surface in which the impeller blade snugly fits.

**15.** A mixed-flow blood pump as defined in claim 11, wherein:

the impeller blade has a constant height.

**16.** A mixed-flow blood pump as defined in claim 14, further comprising:

a flow straightener structure connected to the impeller housing for straightening the blood flow from the impeller blade; and
a flow diffuser structure downstream the flow straightener structure to diffuse the straightened blood flow.


**Patentansprüche**

**1.** Mischströmungs-Blutpumpe (2), die die Merkmale sowohl einer Axialströmungs- als auch einer Radialströmungs-Pumpe aufweist, mit:

einer stationären Gehäusestruktur (22), die eine Längsachse (54), einen axial verlaufenden, ringförmigen Bluteinlasskanal (76), einen radial verlaufenden, ringförmigen Bluteinlasskanal (14), einen axialen Blutauslass (28) und eine axiale Blutleitung zwischen (a) den axial und radial verlaufenden Bluteinlasskanälen und (b) dem axialen Blutauslass definiert; und
einem drehbaren Flügelrad (50), das in der stationären Gehäusestruktur angebracht ist und umfasst:

eine Flügelradwelle (48), die um die Längsachse der stationären Gehäusestruktur drehbar ist und in der axialen Blutleitung einen Wellenabschnitt aufweist, der sich in einer Richtung, die zu der Richtung der Blutströmung entgegengesetzt ist, konisch verjüngt; und
eine Flügelradschaufel (70), die an dem sich konisch verjüngenden Wellenabschnitt angebracht ist;

wobei der axial verlaufende, ringförmige Bluteinlasskanal, der radial verlaufende, ringförmige Bluteinlasskanal, der sich konisch verjüngende Wellenabschnitt, die an dem sich konisch verjüngenden Wellenabschnitt angebrachte Flügelradschaufel und der axiale Blutauslass so beschaffen sind, dass sie die Mischströmungs-Blutpumpe an einem gegebenen Punkt einer Kurve für den maximalen hydraulischen Wirkungsgrad, die eine bestimmte Pumpendrehzahl mit einem bestimmten Pumpendurchmesser in Beziehung setzt, betreiben, wobei sich der gegebene Punkt in einem Übergangsbereich der Kurve für maximalen hydraulischen Wirkungsgrad zwischen der Axialströmungs- und der Radialströmungs-Pumpe befindet.

**2.** Mischströmungs-Blutpumpe nach Anspruch 1, bei der die bestimmte Pumpendrehzahl und der bestimmte Pumpendurchmesser dimensionslose Werte haben.

**3.** Mischströmungs-Blutpumpe nach Anspruch 2, bei der der dimensionslose Wert der bestimmten Pumpendrehzahl 1,62 ist.

**4.** Mischströmungs-Blutpumpe nach Anspruch 1, bei der:

die stationäre Gehäusestruktur einen ersten (12) und einen zweiten (14) ringförmigen Bluteinlass, die axial beabstandet sind, umfasst;
der axial verlaufende, ringförmige Bluteinlasskanal zwischen dem ersten ringförmigen Bluteinlass und der axialen Blutleitung verläuft; und
der radial verlaufende, ringförmige Bluteinlasskanal zwischen dem zweiten ringförmigen Bluteinlass und der axialen Blutleitung verläuft.

**5.** Mischströmungs-Blutpumpe nach Anspruch 4, bei der:

der axial verlaufende, ringförmige Bluteinlasskanal einen im Allgemeinen zylindrischen axialen Kanalabschnitt umfasst.

**6.** Mischströmungs-Blutpumpe nach Anspruch 4, bei der:

der radial verlaufende, ringförmige Bluteinlasskanal in Bezug auf die Längsachse des stationären Gehäuses einen spitzen Winkel definiert.

**7.** Mischströmungs-Blutpumpe nach Anspruch 5, bei der
die stationäre Gehäusestruktur ein zylindrisches Element mit einer inneren Oberfläche aufweist; und
der im Allgemeinen zylindrische axiale Kanalabschnitt zwischen der Flügelradwelle und der inneren Oberfläche des zylindrischen Elements einen Spalt aufweist.

**8.** Mischströmungs-Blutpumpe nach Anspruch 1, bei der die Flügelradschaufel eine an der Flügelradwelle angebrachte Flügelradschaufel des Förderschneckentyps (Auger-Typ) aufweist.

**9.** Mischströmungs-Blutpumpe nach Anspruch 1, bei der:

die stationäre Gehäusestruktur um die Flügelradwelle ein zylindrisches Element aufweist; und
die Mischströmungs-Blutpumpe ferner eine Elektromotorstruktur aufweist, die umfasst:

- Permanentmagneten, die in die Flügelradwelle eingebettet sind; und
- elektrische Windungen, die in dem zylindrischen Element der stationären Gehäusestruktur angebracht sind.

**10.** Mischströmungs-Blutpumpe nach Anspruch 7, wobei:

die Mischströmungs-Blutpumpe ferner im Bereich des Spalts zwischen der Flügelradwelle und der inneren Oberfläche des zylindrischen Elements eine Elektromotorstruktur aufweist, die umfasst:

- Permanentmagneten, die in die Flügelradwelle eingebettet sind, und elektrische Windungen, die in dem zylindrischen Element der stationären Gehäusestruktur angebracht sind.

**11.** Mischströmungs-Blutpumpe nach Anspruch 1, bei der:

die Flügelradwelle ein erstes und ein zweites Ende, die einander gegenüberliegen, und einen ersten und einen zweiten Stirnzapfen, die einander gegenüberliegen, umfasst; und
die stationäre Gehäusestruktur eine erste und eine zweite Buchsenhalterung am ersten bzw. am zweiten Ende der Flügelradwelle aufweist, um den ersten bzw. den zweiten Stirnzapfen, die einander gegenüberliegen, aufzunehmen.

**12.** Mischströmungs-Blutpumpe nach Anspruch 11, bei der:

der erste Zapfen in einer Richtung, die zu der Richtung der Blutströmung entgegengesetzt ist, sich leicht konisch verjüngt und ein freies Ende mit kleinerem Durchmesser aufweist;
die erste Buchsenhalterung eine erste Buchse aufweist, um das freie Ende mit kleinerem Durchmesser des ersten Zapfens aufzunehmen;
die erste Buchsenhalterung im Bereich der ersten Buchse eine konkave, im Allgemeinen halbkugelförmige Oberfläche aufweist; und
das erste Ende der Flügelradwelle konvex und im Allgemeinen halbkugelförmig ist.

**13.** Mischströmungs-Blutpumpe nach Anspruch 11, bei der:

der zweite Zapfen sich in einer Richtung, die zu der Richtung der Blutströmung entgegengesetzt ist, leicht konisch verjüngt und ein freies Ende mit größerem Durchmesser aufweist;
die zweite Buchsenhalterung eine zweite Buchse aufweist, um das freie Ende mit größerem Durchmesser des zweiten Zapfens aufzunehmen;
die zweite Buchsenhalterung im Bereich der zweiten Buchse eine konvexe, im Allgemeinen halbkugelförmige Oberfläche aufweist; und

das zweite Ende der Flügelradwelle im Allgemeinen flach ist.

14. Mischströmungs-Blutpumpe nach Anspruch 1, bei der:

die stationäre Gehäusestruktur ein Flügelradgehäuse um die Flügelradschaufel aufweist, wobei das Flügelradgehäuse eine innere Oberfläche besitzt, in die die Flügelradschaufel eng eingepasst ist.

15. Mischströmungs-Blutpumpe nach Anspruch 11, bei der:

die Flügelradschaufel eine konstante Höhe besitzt.

16. Mischströmungs-Blutpumpe nach Anspruch 14, die ferner umfasst:

eine Strömungsgeraderichtungsstruktur, die mit dem Flügelradgehäuse verbunden ist, um die Blutströmung von der Flügelradschaufel gerade zu richten; und
eine Strömungsumlenkstruktur stromabseitig von der Strömungsgeraderichtungsstruktur, die die gerade gerichtete Blutströmung umlenkt.

## Revendications

1. Une pompe à sang à écoulement mixte (2) présentant des caractéristiques à la fois des pompes à écoulement axial et des pompes à écoulement radial, comprenant:

une structure de boîtier stationnaire (22) définissant un axe longitudinal (54), un passage d'entrée du sang annulaire s'étendant axialement (76), un passage d'entrée du sang annulaire s'étendant radialement (14), une sortie de sang axiale (28), et un conduit de sang axial entre (a) les passages d'entrée du sang s'étendant axialement et radialement, et (b) la sortie de sang axiale; et
une turbine rotative (50) montée dans la structure de boîtier stationnaire, comprenant:

un arbre de turbine (48) rotatif autour de l'axe longitudinal de la structure de boîtier stationnaire, l'arbre de turbine ayant, dans le conduit de sang axial, une portion d'arbre effilée dans une direction opposée à la direction d'écoulement du sang; et
une pale de turbine (70) montée sur la portion d'arbre effilée;

- dans lequel le passage d'entrée du sang annulaire s'étendant axialement, le passage d'entrée du sang annulaire s'étendant radialement, la portion d'arbre effilée, la pale de turbine montée sur la portion d'arbre effilée, et la sortie de sang axiale peuvent faire fonctionner la pompe à sang à écoulement mixte à un point donné d'une courbe d'efficacité hydraulique maximale reliant une vitesse de rotation spécifique de la pompe avec un diamètre spécifique de la pompe, ledit point donné étant situé dans une région de transition de la courbe d'efficacité hydraulique maximale entre les pompes à écoulement axial et les pompes à écoulement radial.

2. Une pompe à sang à écoulement mixte telle que définie dans la revendication 1, dans laquelle la vitesse de rotation spécifique de la pompe et le diamètre spécifique de la pompe ont des valeurs sans dimension.

3. Une pompe à sang à écoulement mixte telle que définie dans la revendication 2, dans laquelle la valeur sans dimension de la vitesse de rotation spécifique de la pompe est 1.62.

4. Une pompe à sang à écoulement mixte telle que définie dans la revendication 1, dans laquelle:

la structure de boîtier stationnaire comporte des première (12) et seconde (13) entrées de sang annulaires espacées axialement;
le passage d'entrée de sang annulaire s'étendant axialement s'étend entre la première entrée de sang annulaire et le conduit de sang axial; et
le passage d'entrée de sang annulaire s'étendant radialement s'étend entre la seconde entrée de sang annulaire et le conduit de sang axial.

5. Une pompe à sang à écoulement mixte telle que définie dans la revendication 4, dans laquelle:

le passage d'entrée de sang annulaire s'étendant axialement comporte une portion de passage axial généralement cylindrique.

6. Une pompe à sang à écoulement mixte telle que définie dans la revendication 4, dans laquelle :

le passage d'entrée de sang annulaire s'étendant radialement définit un angle aigu par rapport à l'axe longitudinal de la structure de boîtier stationnaire.

7. Une pompe à sang à écoulement mixte telle que définie dans la revendication 5, dans laquelle:

la structure de boîtier stationnaire comporte un élément cylindrique avec une surface intérieure; et
la portion de passage axial généralement cylindrique comporte un espacement entre l'arbre de turbine et la surface intérieure de l'élément cylindrique.

8. Une pompe à sang à écoulement mixte telle que définie dans la revendication 1, dans laquelle la pale de turbine comporte un pale de turbine de type vis sans fin montée sur l'arbre de turbine.

9. Une pompe à sang à écoulement mixte telle que définie dans la revendication 1, dans laquelle:

la structure de boîtier stationnaire comporte un élément cylindrique autour de l'arbre de turbine; et
la pompe à sang à écoulement mixte comporte en outre une structure de moteur électrique comprenant :

- des aimants permanents noyés à l'intérieur de l'arbre de turbine; et
- des enroulements électriques montés dans l'élément cylindrique de la structure de boîtier stationnaire.

10. Une pompe à sang à écoulement mixte telle que définie dans la revendication 7, dans laquelle:

la pompe à sang à écoulement mixte comprend en outre, dans la région de l'espacement entre l'arbre de turbine et la surface intérieure de l'élément cylindrique, une structure de moteur électrique comprenant :

- des aimants permanents noyés dans l'arbre de turbine; et des enroulements électriques montés dans l'élément cylindrique de la structure de boîtier stationnaire.

11. Une pompe à sang à écoulement mixte telle que définie dans la revendication 1, dans laquelle:

l'arbre de turbine comporte des première et second extrémités opposées et des premier et second pivots d'extrémités opposées; et
la structure de boîtier stationnaire comprend des premier et second supports de coussinet respectivement aux première et seconde extrémités de l'arbre de turbine pour recevoir les premier et second pivots d'extrémités opposées.

12. Une pompe à sang à écoulement mixte telle que définie dans la revendication 11, dans laquelle:

le premier pivot est légèrement effilé dans une direction opposée à la direction d'écoulement du sang et comprend un extrémité libre de plus petit diamètre;
le premier support de coussinet comprend un premier coussinet pour recevoir l'extrémité libre de plus petit diamètre du premier pivot;
le premier support de coussinet comprend une surface concave généralement hémisphérique dans la région du premier coussinet; et
la première extrémité de l'arbre de turbine est convexe et généralement hémisphérique.

13. Une pompe à sang à écoulement mixte telle que définie dans la revendication 11, dans laquelle:

le second pivot est légèrement effilé dans une direction opposée à la direction d'écoulement de sang et comporte un extrémité libre de plus grand diamètre;
le second support de coussinet comporte un second coussinet pour recevoir l'extrémité libre de plus grand diamètre du second pivot;
le second support de coussinet comprend une surface convexe généralement hémisphérique dans la région du second coussinet; et

la seconde extrémité de l'arbre de turbine est généralement plate.

14. Une pompe à sang à écoulement mixte telle que définie dans la revendication 1, dans laquelle :

la structure de boîtier stationnaire comprend un boîtier de turbine autour de la pale de turbine, le boîtier de turbine ayant une surface intérieure dans laquelle la pale de turbine est ajustée.

15. Une pompe à sang à écoulement mixte telle que définie dans la revendication 11, dans laquelle:

la pale de turbine a une hauteur constante.

16. Une pompe à sang à écoulement mixte telle que définie dans la revendication 1, dans laquelle:

une structure de redresseur d'écoulement reliée au boîtier de turbine pour redresser l'écoulement de sang en provenance de la pale de turbine; et

une structure de diffuseur d'écoulement en aval de la structure de redresseur d'écoulement pour diffuser l'écoulement de sang redressé.

FIG. 1

PELTON TURBINES

CENTRIFUGAL
PUMPS

AXIAL-FLOW
PUMPS

HELIXES

$D_S$

10

1

0.1    1    10

1.62

$N_S$

FIG. 2

EP 1 485 144 B1

FIG. 3

FIG - 4a

FIG-4b

56 · 61 · 60 · 48 · 62 · 72 · 66

Fig. 5a

48 · 65 · 64 · 58 · 68 · 74

Fig. 5b

86

4

2

90

88

94

92

FIG. 6